# EUROPÄISCHE PATENTSCHRIFT

(11) **EP 0 768 068 B1**
(45) Veröffentlichungstag und Bekanntmachung des Hinweises auf die Patenterteilung: **20.06.2001**
(21) Anmeldenummer: 96114831.9
(22) Anmeldetag: 16.09.1996
(51) Int. Cl.: A61F 2/52

(54) **Brustprothese**
Breast prosthesis
Prothèse mammaire

(30) Priorität: 13.10.1995 DE 29516281 U
(43) Veröffentlichungstag der Anmeldung: 16.04.1997
(73) Patentinhaber: AMOENA Medizin-Orthopädie-Technik GmbH, D-83064 Raubling (DE)
(72) Erfinder: Wild, Helmut, D-83115 Neubeuern (DE)
(74) Vertreter: Laufhütte, Dieter, Dr.-Ing.

(56) Entgegenhaltungen:
- EP-A- 0 320 590
- EP-A- 0 681 814
- DE-U- 9 213 880
- FR-A- 2 487 191
- GB-A- 2 262 041

## Beschreibung

Die Erfindung betrifft eine Brustprothese, bestehend aus einem schalenförmigen Körper aus einem weich-elastisch eingestellten Kunststoff, vorzugsweise einer additionsvernetzten Zwei-Komponenten-Silikon-Kautschuk-Masse, der in Kunststoffolien eingeschweißt ist und so eine erste Kammer bildet, und ein aus einer der Trägerin während des Tragens zugewandten und sich an die erste Kammer anschließenden mit einem anderen Füllmaterial gefüllten zweiten Kammer.

Brustprothesen, die aus einem schalenförmigen Körper aus einem weich-elastisch eingestellten additionsvernetzten Zwei-Komponenten-Silikon-Kautschuk bestehen, der in Kunststoffolien eingeschweißt ist, sind beispielsweise schon aus der DE 90 10 426.9 bekannt.

Derartige Brustprothesen werden aus kosmetischen Gründen nach krebsbedingten Brustamputationen aus kosmetischen Gründen eingesetzt. In jüngster Zeit setzen sich neue Operationsmethoden durch, bei welchen nur noch die krebsbefallenen Teile der Brust operiert werden, hier also nur noch eine Teilamputation vorgenommen wird. Hierdurch bedingt entstehen individuell unterschiedlich unregelmäßige Narbenbereiche, die - ebenso wie das stehengebliebene Restgewebe - als solche undefinierte Oberflächen bilden. Beim Tragen herkömmlicher Brustprothesen entsteht nun durch diese unregelmäßige Oberfläche das Problem, daß häufig der weich-elastisch eingestellte Kunststoff aufgrund seiner Eigenelastizität den Narbenbereich der nur teilamputierten Brust beaufschlagt und dadurch zu einer Verringerung des Tragekomforts führt. Bei entsprechend großem Restgewebebestand paßt die Brustprothese häufig nicht mehr.

Aus der DE 44 21 516 A ist eine gattungsgemäße Zwei-Kammer-Brustprothese bekannt, bei der die zweite Kammer mit einem Polsterkörper aus Schaumkunststoff oder einem Faserknäuel gefüllt ist.

EP 0 320 590 A offenbart eine Brustprothese und bildet den ersten Teil des Anspruchs 1.

Aufgabe der vorliegenden Erfindung ist es, eine Brustprothese an die Hand zu geben, die sich insbesondere auch zur Versorgung von Patientinnen nach einer Teilamputation einer Brust eignet.

Erfindungsgemäß wird diese Aufgabe ausgehend von einer gattungsgemäßen Brustprothese dadurch gelöst, daß das Füllmaterial der zweiten Kammer aus einem thixotropen Material besteht, das sich an die unebene Narbensituation der teilamputierten Brust beim Anlegen der Brustprothese anpaßt und diese Form im wesentlichen beibehält.

Besonders vorteilhaft ist ein pastöses Material, das im wesentlichen aus einer Mischung aus nicht vernetztem Silikonöl mit Kieselsäure besteht. Dabei können der Mischung Leichtfüllstoffe, vorzugsweise Kunststoff-Microspheres, beigemengt sein.

Eine besondere Ausgestaltung der Erfindung liegt darin, daß die ebenfalls aus einer Kunststoffolie bestehende Außenwand der zweiten Kammer ein Füllventil aufweist, über welches das Füllmaterial einfüllbar ist. Hierdurch kann individuell durch ein eventuelles Teilbefüllen oder ein entsprechend angepaßtes Befüllen der zweiten Kammer ein entsprechender Zwischenraum geschaffen werden, der eine bessere Anpassung der Brustprothese an die Operationsnarbe schafft.

Die schalenförmige erste Kammer kann mit ihrem Randbereich über den Rand der zweiten Kammer hinausragen, so daß sich eine Höhlung ergibt. Andererseits kann der Rand der zweiten Kammer auf dem Rand der ersten Kammer aufliegen, so daß sich nur eine im Schnitt leicht konkave Wölbung ergibt.

Bei Bedarf kann die Brustprothese als Teilprothese ausgeführt sein, die nur dem zu ersetzenden Brustteil entspricht.

Weitere Einzelheiten und Vorteile der Erfindung werden nachstehend anhand einem in der Zeichnung dargestellten Ausführungsbeispiel näher erläutert. In dieser zeigen:
- Fig.1:: eine schematische Schnittdarstellung einer ersten Ausführungsform der erfindungsgemäßen Brustprothese,
- Fig.2:: einen Schnitt durch eine zweite Ausführungsform einer erfindungsgemäßen Brustprothese,
- Fig.3:: eine Rückansicht der Ausführungsform gemäß Fig.2 vollständig gefüllter zweiter Kammer und
- Fig.4:: eine Rückansicht wie in Fig.3, jedoch mit teilbefüllter zweiter Kammer.

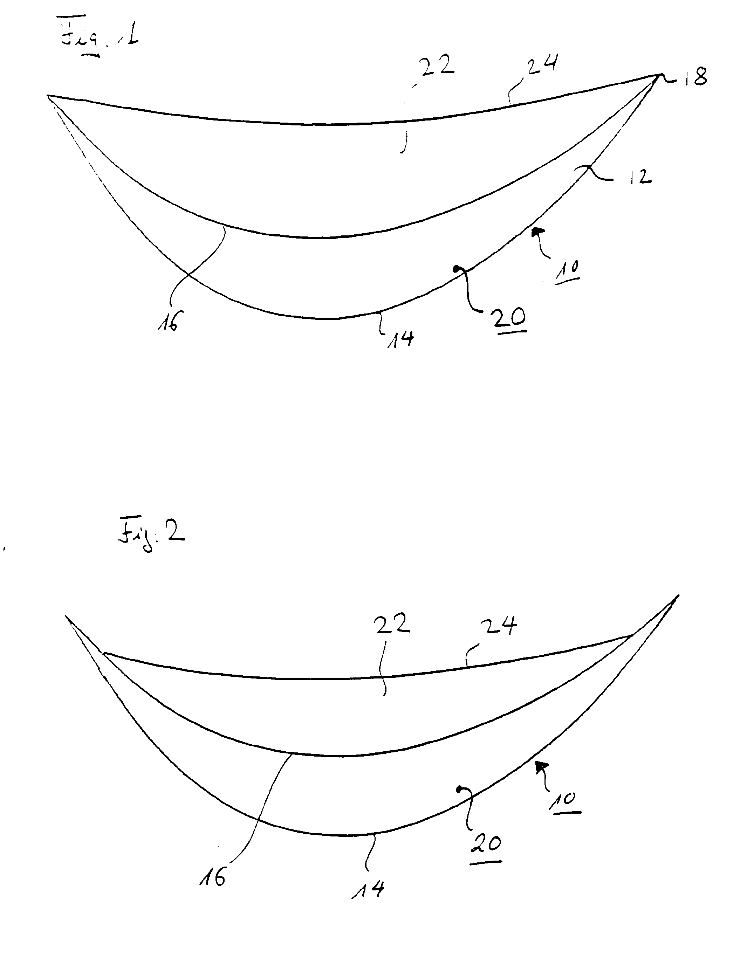

Die in Fig.1 dargestellte Brustprothese 10 besteht aus einem schalenförmigen Körper 12 aus einer weich-elastisch eingestellten additionsvernetzten Zwei-Komponenten-Silikon-Kautschuk-Masse, dessen Außenseite durch eine Polyurethanfolie 14 und dessen Innenseite durch eine Polyurethanfolie 16 abgedeckt sind, die längs ihres gemeinsamen Umfangrandes 18 durch eine umlaufende Schweißnaht miteinander verschweißt sind. Hierdurch entsteht eine erste Kammer 20. Innerhalb der durch die schalenförmige Ausbildung der ersten Kammer 20 bedingten Höhlung ist eine zweite Kammer 22 angeordnet, die durch eine ebenfalls als Polyurethanfolie ausgebildete Wandung 24 begrenzt ist. Die Polyurethanwandungen 14, 16 und 24 weisen in dem Ausführungsbeispiel gemäß Fig.1 vorteilhaft einen gemeinsamen Umfangsrand 18, der als gemeinsame Schweißnaht ausgebildet ist, auf. In der zweiten Kammer 22 ist erfindungsgemäß ein pastöses Material eingefüllt, das aus einer Mischung aus nicht vernetztem Silikonöl mit Kieselsäure besteht, den Kunststoff-Microspheres als Leichtfüllstoff beigemengt ist. Die Mischung aus Silikonöl und Kieselsäure eignet sich besonders gut als pastöses Material, da eine Verschieblichkeit und damit Anpassung an den Narbenbereich durch das Silikonöl gewährleistet ist, während andererseits die Kieselsäureteilchen dazu bestrebt sind, miteinander zu verhaken und daher eine weitgehend formstabile Einheit zu bilden, soweit hier nicht weiterer Druck auf die Oberfläche der zweiten Kammer ausgeübt wird. Die Kunststoff-Microspheres dienen dazu, das spezifische Gewicht der Gesamtmischung zu verringern und die gesamte Brustprothese dadurch leichter zu machen.

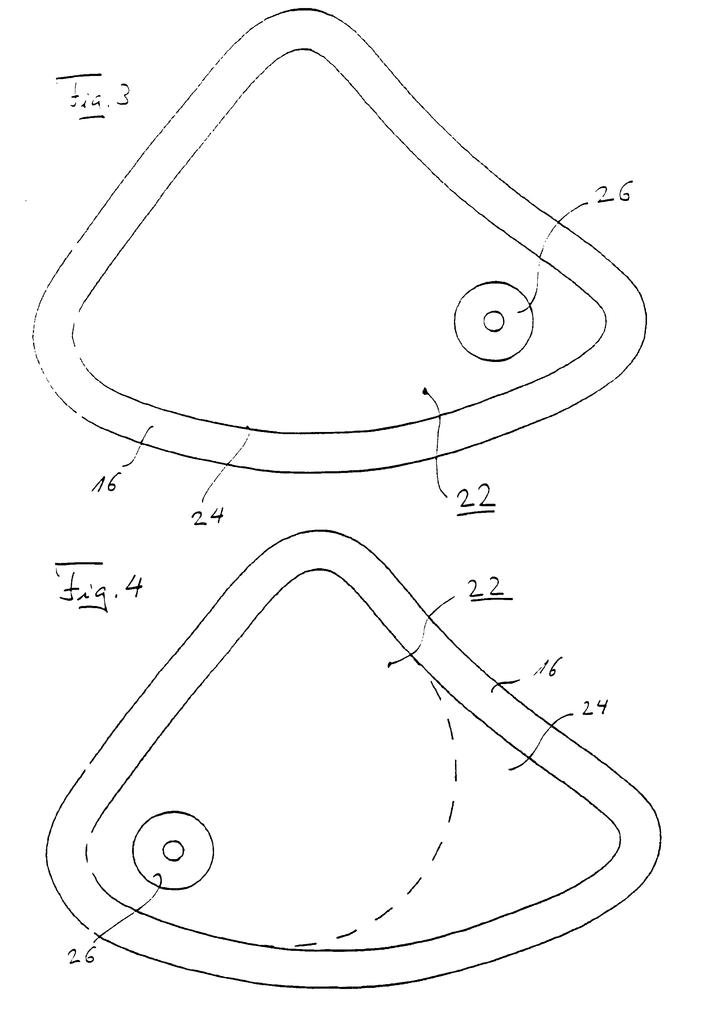

In Fig.2 ist eine abgeänderte Form einer erfindungsgemäßen Brustprothese 10 gezeigt, bei der der Randbereich der ersten Kammer über den Rand der zweiten Kammer hinausragt. Hier wird also eine flache Höhlung gebildet, wie sie sich aus dem Schnitt in Fig.2 ergibt. In der Rückansicht gemäß Fig.3 wird deutlich, daß in der Kunststoffolie 24 ein Einfüllventil 26 integriert ist. Über dieses Einfüllventil kann das pastöse Material in die zweite Kammer 22 eingefüllt werden. Dabei kann über die eingefüllte Menge eine optimale Anpassung der Oberfläche der zweiten Kammer an den Narbenbereich der teilamputierten zweiten Brust vorgesehen werden.

Vorteilhaft kann beispielsweise eine Brustprothese mit gar nicht oder nur teilbefüllter zweiter Kammer in den Handel abgegeben werden, wobei dort die Prothese durch entsprechendes Auffüllen der zusätzlichen Kammer mit dem pastösen Material an die aktuelle Narbensituation der Trägerin angepaßt werden kann. Durch das erfindungsgemäße Füllmaterial für die zweite Kammer ist es nun gewährleistet, daß sich diese beim Auffüllen mit dem pastösen Material an die Narbensituation anpaßt und in dieser Stellung formstabil verbleibt. Bei Veränderung der Narbensituation kann die Prothese ohne weiteres an die neue Umgebung angepaßt werden und verbleibt ebenfalls nach Annahme dieser neuen Form formstabil. In der Darstellung gemäß Fig.4 ist eine nur teilbefüllte zweite Kammer gezeigt, wobei über die gestrichelte Linie der Grad der Teilbefüllung angedeutet ist.

## Patentansprüche

1. Brustprothese (10), bestehend aus einem schalenförmigen Körper (12) aus einem weich-elastisch eingestellten Kunststoff, vorzugsweise einer additionsvernetzten Zwei-Komponenten-Silikon-Kautschuk-Masse, der in Kunststoffolien eingeschweißt ist und so eine erste Kammer (20) bildet, und einer der Trägerin während des Tragens zugewandten und sich an die erste Kammer anschließenden mit einem anderen Füllmaterial gefüllten zweiten Kammer (22),
dadurch gekennzeichnet,
daß das Füllmaterial der zweiten Kammer (22) aus einem thixotropen Material besteht.

2. Brustprothese nach Anspruch 1, dadurch gekennzeichnet, daß das Füllmaterial der zweiten Kammer im wesentlichen aus einer Mischung aus nicht vernetztem Silikonöl mit Kieselsäure besteht.

3. Brustprothese nach Anspruch 2, dadurch gekennzeichnet, daß der Mischung aus nicht vernetztem Silikonöl mit Kieselsäure Leichtfüllstoffe, vorzugsweise Kunststoff-Microspheres, beigemengt sind.

4. Brustprothese nach einem der Ansprüche 1 bis 3, dadurch gekennzeichnet, daß die ebenfalls aus einer Kunststoffolie bestehende Außenwand der zweiten Kammer ein Füllventil aufweist, über welches das Füllmaterial einfüllbar ist.

5. Brustprothese nach einem der Ansprüche 1 bis 4, dadurch gekennzeichnet, daß die schalenförmige erste Kammer mit ihrem Randbereich über den Rand der zweiten Kammer hinausragt.

6. Brustprothese nach einem der Ansprüche 1 bis 4, dadurch gekennzeichnet, daß der Rand der zweiten Kammer auf dem Rand der ersten Kammer liegt.

7. Brustprothese nach einem der Ansprüche 1 bis 6, dadurch gekennzeichnet, daß sie als Teilprothese ausgeführt ist.

## Claims

1. Mammary prosthesis (10) consisting of a shell-like body (12) of a plastic with flexible elastic properties, preferably an addition-crosslinked two-component silicone rubber compound, which is welded into plastic sheets so forming a first chamber (20), and a second chamber (22) turned towards the bearer while being worn, that is connected to the first chamber and is filled with another filler material,
characterized in
that the filler material of the second chamber (22) consists of a thixotropic material.

2. The mammary prosthesis as claimed in claim 1, characterized in that the filler material of the second chamber substantially consists of a mixture of non-crosslinked silicone oil with silicic acid.

3. The mammary prosthesis as claimed in claim 2, characterized in that light filler materials, preferably plastic microspheres, are added to the mixture of non-crosslinked silicone oil with silicic acid.

4. The mammary prosthesis as claimed in any of claims 1 to 3, characterized in that the outer wall of the second chamber, likewise consisting of a plastic sheet, has an inlet valve via which the filler material can be introduced.

5. The mammary prosthesis as claimed in any of claims 1 to 4, characterized in that the edge zone of the shell-like first chamber extends beyond the edge of the second chamber.

6. The mammary prosthesis as claimed in any of claims 1 to 4, characterized in that the edge of the second chamber lies on the edge of the first chamber.

7. The mammary prosthesis as claimed in any of claims 1 to 6, characterized in that it is designed as a partial prosthesis.

## Revendications

1. Prothèse mammaire (10) consistant en un corps en forme de coque (12) en un matériau plastique présentant des propriétés molles élastiques, de préférence une masse à deux composants silicone/caoutchouc réticulée par addition, qui est soudé dans des films en plastique et qui forme ainsi une première chambre (20), et une seconde chambre (22) qui est dirigée vers la porteuse pendant qu'elle porte la prothèse et qui est contiguë à la première chambre et est remplie d'un autre matériel de remplissage,
caractérisée en ce que
le matériel de remplissage de la seconde chambre (22) consiste en un matériel thixotrope.

2. Prothèse mammaire selon la revendication 1, caractérisée en ce que le matériel de remplissage de la seconde chambre consiste essentiellement en un mélange d'huile de silicone non-réticulée et d'acide silique.

3. Prothèse mammaire selon la revendication 2, caractérisée en ce que le mélange d'huile de silicone non-réticulée et d'acide silique est additionné de matériels de remplissage légers, de préférence de microsphères plastiques.

4. Prothèse mammaire selon l'une quelconque des revendications 1 à 3, caractérisée en ce que la paroi extérieure de la seconde chambre qui consiste également en un film plastique présente une soupape de remplissage par laquelle le matériel de remplissage peut être rempli.

5. Prothèse mammaire selon l'une quelconque des revendications 1 à 4, caractérisée en ce que la première chambre en forme de coque dépasse par sa zone de bordure le bord de la seconde chambre.

6. Prothèse mammaire selon l'une quelconque des revendications 1 à 4, caractérisée en ce que le bord de la seconde chambre repose sur le bord de la première chambre.

7. Prothèse mammaire selon l'une quelconque des revendications 1 à 6, caractérisée en ce qu'elle est exécutée comme prothèse partielle.
